# EUROPEAN PATENT APPLICATION

(11) **EP 2 821 396 A1**
(43) Date of publication of application: **07.01.2015**
(21) Application number: 13754169.4
(22) Date of filing: 26.02.2013
(51) Int. Cl.: C07C 319/28, C07C 323/58

(54) **METHOD FOR MANUFACTURING REFINED METHIONINE**

(30) Priority: 27.02.2012 JP 2012040553
(71) Applicant: Sumitomo Chemical Company Limited, Tokyo 104-8260 (JP)
(72) Inventor: NISHIDA, Junichi, 08007 Barcelona (ES); KOIZUMI, Yoshiyuki, Niihama-shi Ehime 792-8521 (JP)
(74) Representative: Vossius & Partner
(86) International application number: PCT/JP2013/054982
(87) International publication number: WO 2013/129405

(57) **Abstract**

The object of the present invention is to provide a process for producing refined methionine in which the loss of methionine due to washing is reduced.

The present invention relates to a process for producing refined methionine from crude methionine, comprising a step of washing crude methionine with the use of a wash water containing methionine.

## Description

### Technical Field

The present invention relates to a process for producing a refined methionine from crude methionine, in particular a process for producing refined methionine in high yield by reducing the loss of methionine due to washing. Methionine is useful as an additive for animal feed.

### Background Art

Patent Document 1 discloses a process for producing methionine in which 5-(β-methylmercaptethyl) hydantoin is hydrolyzed in the presence of alkali compound such as potassium carbonate and sodium hydroxide, then the hydrolyzed solution is neutralized by adding an acid such as carbon dioxide gas or sulfuric acid. Solid-liquid separation is carried out after crystallization of methionine, then resulting crude methionine is washed and dried to obtain refined methionine. In addition, Patent Document 2 discloses that crystalline methionine produced in fermentation by a methionine-producing-microorganism is separated, and then, the crystal is washed.

Since the crystalized methionine in the process is in a slurry state and a methionine cake separated by solid-liquid separation from the slurry comprises impurities, washing is usually performed and water is generally used for washing.

On the other hand, a process in which the filtrate is condensed and circulated in the hydrolyzing step of hydantoin is known.

### Prior Art Document

### Patent Document

Patent Document 1: JP 2000-143617 A
Patent Document 2: JP 2007-514430 A

### Summary of Invention

### Problems to be solved by Invention

Since methionine dissolves in water in the range including the saturated solubility as an upper limit, washing of a cake of methionine with water leads to the loss of methionine contained in the cake.
Although the use of cold water is conceivable so as to reduce the solubility of methionine, it leads to an increase of energy consumption.

An object of the present invention is to provide a process for producing refined methionine in which the loss of methionine due to washing is reduced.

### Means for solving Problems

The present inventors have intensively made researches, as a result, they have found that washing of a cake of methionine (hereinafter, also referred to as "crude methionine") which is separated by solid-liquid separation from a slurry, with the use of a wash water containing methionine (hereinafter, also referred to as "methionine-containing wash water") reduces the amount of methionine dissolved from the cake and completed the present invention.

In addition, the present inventors have found that the methionine-containing wash water after being used for the washing can be recovered and reused.

Furthermore, the present inventors have found that the quality of refined methionine, which is obtained by washing crude methionine with the reuse of the methionine-containing wash water recovered after being used for washing, is the same as that of refined methionine obtained by washing with the use of pure water.

That is, the present invention is as follows.
[1] A process for producing refined methionine from crude methionine, comprising a step of washing crude methionine with the use of a wash water containing methionine.
[2] The process according to [1], wherein the concentration of methionine in the wash water is 1.0% by weight or higher.
[3] The process according to [1] or [2], wherein the wash water after being used for washing crude methionine is recovered and reused.
[4] The process according to [3], wherein washing of crude methionine is performed with the use of an additional wash water together with the reuse of a part of the wash water after being used for washing crude methionine.
[5] The process according to [4], wherein washing of crude methionine is performed by washing with the reused wash water, and then, washing with the additional wash water.
[6] The process according to [4] or [5], wherein the additional wash water contains methionine.
[7] The process according to [6], wherein the concentration of methionine in the additional wash water is 2.0% by weight or higher.
[8] The process according to [6], wherein the additional wash water is substantially free from alkali.
[9] The process according to any one of [6] to [8], further comprising a step of drying refined methionine, wherein the additional wash water is prepared by dissolving dried and refined methionine obtained in the step in pure water.

### Effect of Invention

According to the present invention, it is possible to reduce the loss of methionine due to washing since washing of crude methionine is performed with the use of a methionine-containing wash water, and thus, it is possible to increase the yield of refined methionine.

In addition, the methionine-containing wash water after being used for washing crude methionine is recovered and reused, and thus, methionine contained in the wash water is efficiently utilized as methionine for a wash water. There is no need for wastewater treatment. Furthermore, the amount of a methionine-containing wash water which is newly prepared is reduced. These lead to cost saving.

Furthermore, the process can provide refined methionine in high quality since the quality of refined methionine, which is obtained by washing crude methionine with the reuse of the recovered and methionine-containing wash water, is equal to that of refined methionine obtained by washing with the use of pure water.

### Brief Description of Drawings

Figure 1 is a block diagram of a preferable embodiment of steps (washing step and drying step) for producing refined methionine from crude methionine according to the present invention.
Figure 2 shows a washing apparatus used in Example and Comparative Example.

### Mode for carrying out Invention

The process of the present invention for producing refined methionine comprises a step of washing crude methionine with a methionine-containing wash water. Although crude methionine used in the process of the present invention is not specifically limited, the process of the present invention is preferable as a process for producing refined methionine from crude methionine which is obtained from hydrolysis reaction of 5-(2-(methylthio)ethyl)imidazolidine-2,4-dione [see following formula (1)].

For example, 5-[2-(methylthio)ethyl] imidazolidine-2,4-dione is used as a raw material and hydrolyzed in the presence of an alkali compound (hydrolyzing step) to obtain a reaction liquid containing methionine as an alkali salt, then, crude methionine can be obtained by taking methionine out from the reaction liquid.

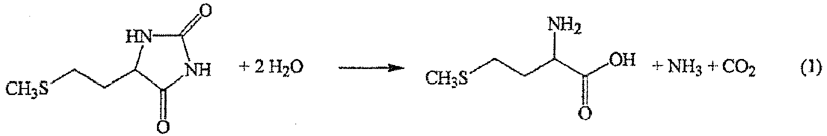

5-[2-(methylthio)ethyl] imidazolidine-2,4-dione which is used as a raw material can be prepared, for example, by reacting 2-hydroxy-4-methylthio butanenitrile with ammonia and carbon dioxide, or, with ammonium carbonate (see following reaction formulas (2) and (3)).

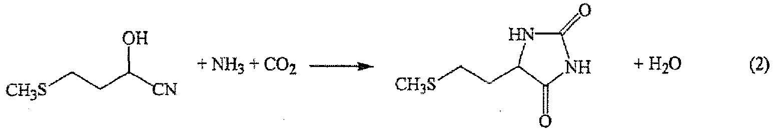

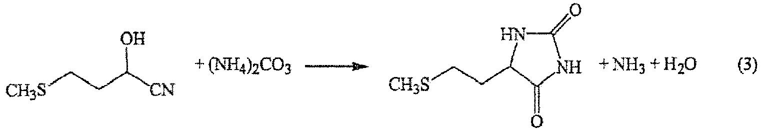

Examples of the alkali compound include, for example, potassium hydroxide, sodium hydroxide, potassium carbonate, potassium hydrogen carbonate. Two or more kinds of the alkali compound may be used as necessary. The use amount of the alkali compound as the amount of potassium or sodium is usually from 2 to 10 equivalent, preferably from 3 to 6 equivalent relative to 1 equivalent of 5-[2-(methylthio) ethyl] imidazolidine-2,4-dione. The use amount of water is usually from 2 to 20 times by weight relative to 5-[2-(methylthio)ethyl]imidazolidine-2,4-dione.

Hydrolysis reaction may be carried out in a stirring type or non-stirring type, and continuous or discontinuous (batch type) reaction tank. However, hydrolysis reaction is preferably carried out in a non-stirring type continuous reaction tank in view of the property of the liquid and reactivity.

Hydrolysis reaction is preferably carried out under increased pressure of about 0.5 to 1 MPa (gauge pressure) with heating at a temperature of about 150 to 200 °C. The reaction time is usually from 10 minutes to 24 hours.

Crude methionine can be obtained as follows: crystallization is carried out by, for example, introducing carbon dioxide into the resulting hydrolysis reaction liquid, and then, solid-liquid separation of the obtained slurry is carried out to take methionine out. Solid-liquid separation may be carried out by filtration, decantation or centrifugal separation etc. However, solid-liquid separation is preferably carried out by centrifugal separation.

By introducing carbon dioxide, carbon dioxide is absorbed in the hydrolysis reaction liquid, and then, an alkali salt of methionine precipitates as free methionine.

Carbon dioxide is preferably introduced under increased pressure of usually from 0.1 to 1 MPa and preferably from 0.2 to 0.5 MPa (gauge pressure).

The crystallization temperature is usually from 0 to 50°C and preferably from 10 to 30°C. In addition, although the time until the hydrolysis reaction liquid is saturated with carbon dioxide and methionine sufficiently precipitates may be regarded as a standard time for the crystallization time, the crystallization time is usually from 30 minutes to 24 hours.

Since obtained crude methionine comprises alkali compounds used for hydrolysis reaction, methionine dimer, and impurities such as glycine and alanine produced by the decomposition of methionine, it is necessary to remove these components by washing.

Up to now, said washing has been carried out with the use of water. Washing with the use of water results in a loss of dissolved methionine since the solubility of methionine in pure water is about 3.0% by weight at normal temperature and normal pressure.

In the present invention, washing of crude methionine is carried out with the use of a methionine-containing wash water. This enables to reduce the loss of methionine due to washing and increase the yield of refined methionine.

The concentration of methionine in the methionine-containing wash water is preferably 1.0% by weight or higher and more preferably 2.0% by weight or higher in view of reducing the loss of methionine. The upper limit of the concentration is the saturated solubility (the saturated solubility in pure water is about 3.0% by weight at normal temperature and normal pressure).

The use amount of the methionine-containing wash water is preferably from 100 to 300 g and more preferably from 150 to 250 g relative to 100 g of crude methionine in view that alkali compounds and impurities can be sufficiently removed.

The washing process may be a process in which the methionine-containing wash water is sprayed from a nozzle to crude methionine, a process in which the methionine-containing wash water is added to crude methionine and they are mixed, or the like. In the present invention, the process in which the methionine-containing wash water is sprayed from a nozzle to crude methionine is preferable in view of time efficiency.

After washing, the methionine-containing wash water is removed by a process of centrifugal separation, filtration or the like. The methionine-containing wash water is preferably removed by a process of centrifugal separation in view of that separation can be efficiently carried out.

Washing may be carried out only one time, or may also be carried out a plurality of times. Washing and removal of the methionine-containing wash water are preferably carried out in the same reaction tank, preferably in a noncontinuous (batch type) reaction tank.

After washing, the removed and methionine-containing wash water may be exposed to wastewater treatment. In view of cost saving, the removed and methionine-containing wash water is preferably recovered and reused as a methionine-containing wash water (hereinafter, the methionine-containing wash water, which is recovered after washing of crude methionine and reused, is also referred to as "methionine-containing reused wash water"). By means of this, methionine contained in the reused wash water can be efficiently utilized as methionine for a wash water; there is no need for wastewater treatment; furthermore, the amount of a methionine-containing wash water which is newly prepared is reduced; and these lead to cost saving.

The methionine-containing recovered wash water may be reused in their entirety or in part.

Generally, the solubility of methionine increases under alkaline condition. When crude methionine obtained through a hydrolysis process is washed, the methionine-containing recovered wash water comprises alkali compounds used in the hydrolysis reaction, and thus, the solubility of methionine in the wash water increases. In addition, the concentration of methionine in the methionine-containing recovered wash water increases by the amount equal to the amount of methionine dissolved from crude methionine, in comparison to the concentration of the wash water before being used for washing.

Washing of crude methionine may be performed with the use of only the methionine-containing recovered wash water. In view of product quality, washing is preferably performed with the use of an additional wash water together with the reuse of a part of the methionine-containing recovered wash water. In this case, washing of crude methionine is preferably performed by washing with the methionine-containing recovered wash water, followed by the washing with the additional wash water in view of the concentration of impurities remaining after washing.

In the present invention, the term "wash water" means water used for washing crude methionine. The additional wash water may be pure water. In order to further reduce the loss of methionine, the additional wash water is preferably a methionine-containing wash water which is newly prepared (hereinafter, also referred to as "methionine-containing additional wash water").

The concentration of methionine in the methionine-containing additional wash water which is newly prepared is preferably 2.0% by weight or higher and more preferably 2.5% by weight or higher in view of reducing the loss of methionine. The upper limit of the concentration is the saturated solubility (the saturated solubility in pure water is about 3.0% by weight at normal temperature and normal pressure).

The methionine-containing additional wash water which is newly prepared is preferably prepared from pure water and methionine and is preferably and substantially free from alkali. In this regard, the term "substantially" means that the content of alkali in the wash water is 0.01% by weight or less and preferably 0.005% by weight or less. In addition, the foregoing methionine is preferably refined methionine and is preferably and substantially free from alkali.

The preparation of the methionine-containing wash water can be carried out by dissolving a certain amount of methionine in pure water. The dissolving temperature is usually from 5 to 35°C.

In addition, the concentration of methionine in the methionine-containing additional wash water which is newly prepared is preferably lower than the concentration of methionine in the methionine-containing reused wash water. As described above, the concentration of the methionine-containing recovered wash water increases by the amount equal to the amount of methionine dissolved from crude methionine, in comparison to that before being used for washing. If the concentration of methionine in the methionine-containing additional wash water which is newly prepared is lower than the concentration of methionine in the methionine-containing reused wash water, the increase of the concentration of methionine in the methionine-containing recovered wash water can be suppressed even if washing and recovering/reusing are repeated.

Therefore, in case where washing and recovering/reusing are repeated, the concentration of methionine and the concentration of alkali can be maintained nearly constant by the selection of the use amount of the methionine-containing reused wash water, the concentration of methionine in the methionine-containing reused wash water, the use amount of the methionine-containing additional wash water which is newly prepared, and the concentration of methionine in the methionine-containing additional wash water.

In the present invention, the concentration of methionine in the methionine-containing reused wash water is preferably 3.0% by weight or higher and more preferably 4.0% by weight or higher. It is noted that, since the methionine-containing recovered wash water is alkaline, the saturated solubility of methionine increases in comparison to the solubility in pure water (for example, about 5.0% by weight under 2.0% by weight of concentration of potassium).

The alkali concentration in the methionine-containing reused wash water is preferably from 0.5 to 4.0% by weight and more preferably from 1.0 to 3.0% by weight.

Then, washed methionine is dried. The drying temperature is usually from 50 to 130°C and preferably from 70 to 110°C. The drying time is usually from 1 to 10 hours and preferably from 2 to 7 hours.

Refined methionine obtained in such a manner has 95% purity or more and preferably 98% purity or more.

It is noted that the purity of refined methionine, which is obtained by washing crude methionine with the reuse of the methionine-containing recovered wash water, is equal to that of refined methionine obtained by washing with the use of pure water.

A part of refined methionine obtained here may be used as methionine for the methionine-containing wash water (in particular, the methionine-containing additional wash water which is newly prepared).

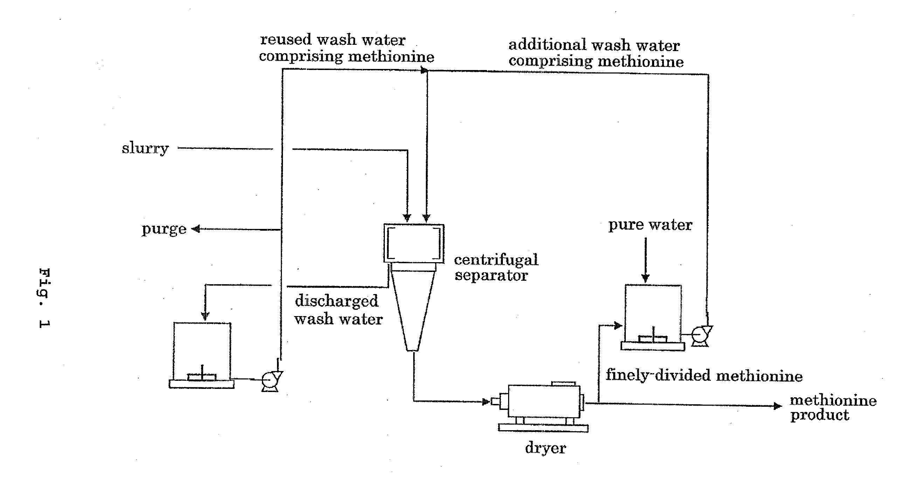

A preferable embodiment of steps (washing step and drying step) for producing crude methionine according to the present invention is shown in Figure 1 as a block diagram. In this regard, arrows indicate flow.

Based on Figure 1, an embodiment in which the methionine-containing wash water recovered after being used for washing crude methionine is reused as a wash water for washing crude methionine is described below.

Crystallization is performed by introducing carbon dioxide into the hydrolysis reaction liquid and the resulting slurry is delivered to a centrifugal separator. Solid-liquid separation is carried out by centrifugal separation to obtain crude methionine. Centrifugal separation is carried out here at from 500 to 5000 rotations per minute and preferably from 1000 to 4000 rotations per minute, for from 1 to 30 minutes and preferably for from 2 to 20 minutes.

Then, washing of crude methionine is performed.

Firstly, crude methionine sticking to the inner of the centrifugal separator is washed with the methionine-containing reused wash water.

The concentration of methionine in the methionine-containing reused wash water is preferably from 3.0 to 5.0% by weight and more preferably from 4.0 to 5.0% by weight, and the alkali concentration is preferably from 0.5 to 4.0% by weight and more preferably from 1.0 to 3.0% by weight.

The use amount of the methionine-containing reused wash water is preferably from 50 to 200 g and more preferably from 70 to 180 g relative to 100 g of crude methionine.

Washing is performed by spraying with the use of nozzle. In this case, the centrifugal separator may rotate at a certain speed and a tip of the nozzle may also rotate so that washing is performed without unevenness. After washing is finished, the wash water is removed by performing centrifugal separation at from 500 to 5000 rotations per minute and preferably from 1000 to 4000 rotations per minute for from 1 to 30 minutes and preferably for from 2 to 20 minutes.

Then, crude methionine sticking to the inner of the centrifugal separator is washed with an methionine-containing additional wash water which is separately and newly prepared.

Methionine used for the methionine-containing additional wash water is preferably refined methionine, and in the present embodiment, methionine used for the methionine-containing additional wash water is refined methionine which is obtained from the drying step described below after this washing step.

The methionine-containing additional wash water is preferably prepared by dissolving refined methionine in pure water. The dissolving temperature is usually from 5 to 35°C.

The concentration of methionine in the methionine-containing additional wash water is preferably from 2.0 to 3.0% by weight and more preferably from 2.5 to 3.0% by weight. The methionine-containing additional wash water is substantially free from alkali and the alkali concentration is preferably 0.01% by weight or lower and more preferably 0.005% by weight or lower.

The use amount of the methionine-containing additional wash water is preferably from 30 to 100 g and more preferably from 50 to 90 g relative to 100 g of crude methionine.

Washing is performed by spraying with the use of a nozzle. In this case, the centrifugal separator may rotate at a certain speed and a tip of the nozzle may also rotate so that washing is performed without unevenness.

After washing is finished, the said wash water is removed by performing centrifugal separation at from 500 to 5000 rotations per minute and preferably from 1000 to 4000 rotations per minute for from 1 to 30 minutes and preferably for from 2 to 20 minutes.

After the wash water is removed, methionine is delivered to a drying machine and dried to obtain refined methionine. The drying temperature is usually from 50 to 130°C and preferably from 70 to 110°C. The drying time is usually from 1 to 10 hours and preferably from 2 to 7 hours.

A part of refined methionine obtained here may be used as methionine for the methionine-containing additional wash water which is newly prepared.

### Examples

Hereinafter, Examples of the present invention is described, which do not limit the scope of the present invention. In Examples, % and part used for describing the concentration or the use amount are based on weight unless otherwise particularly described.

### Reference Example 1

8 parts by weight of potassium carbonate was added to 100 parts by weight of aqueous solution comprising 19% by weight of 5-[2-(methylthio) ethyl]imidazolidine-2,4-dione, then hydrolysis reaction was carried out at a temperature of 180°C under a pressure of 1.0 MPa for 50 minutes to obtain a hydrolysis reaction liquid. To this liquid, carbon dioxide was absorbed at a temperature of 15°C under a pressure of 0.45 MPa for 2 hours to obtain a methionine slurry. 600 g of the obtained methionine slurry was poured into a centrifugal filter rotating 1700 per minute at a pouring speed of 600 g per minute to stick crude methionine on the filter fabric. Then, the rotating speed was set to 3800 per minute and water was thrown off for 2 minutes. At this stage, crude methionine was taken out and the content of pure methionine in crude methionine was determined as 49.0 g (conversion based on HPLC measurement).

### Reference Example 2

A reused wash water was prepared as described below. That is, 117.2 g of a discharged wash water was obtained by washing 49.6 g of crude methionine with 114.6 g of the additional wash water described in Table 2, and then, 87.5 g of the obtained 117.2 g of discharged wash water was mixed with 12.2 g of the additional wash water described in Table 2 and the mixture was used as reused wash water A. Then, 49.6 g of crude methionine was washed with 74.0 g of reused wash water A to obtain a discharged wash water. The total amount of the discharged wash water was mixed with 41.0 g of a discharged wash water which was obtained by further washing the crude methionine with 41.0 g of the additional wash water shown in Table 2. The mixed and discharged wash water (87.5 g) was mixed with the additional wash water (12.2 g) shown in Table 2 and the mixture was used as reused wash water B. Reused wash water B having a formulation shown in Table 1 was obtained by repeating the process 5 times.

### Example

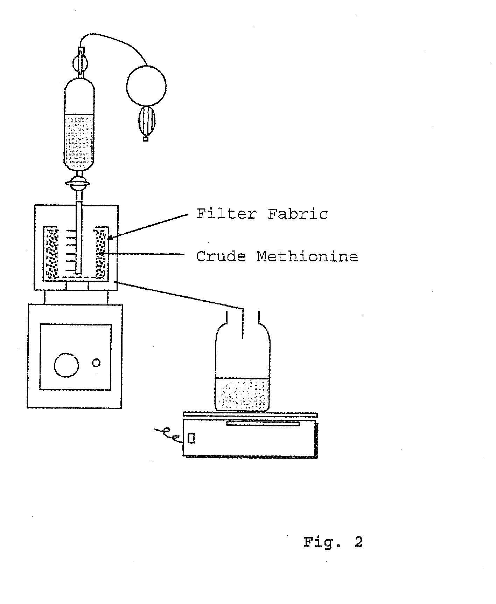

As shown in Figure 2, refined methionine was obtained by washing a cake with a wash water which was sprayed from a nozzle to the cake layer of crude methionine (the formulation is shown in Table 3) sticking to the filter fabric of centrifugal filter (KOKUSAN Co. Ltd. H-112) by a process described below.

600 g of slurry comprising methionine prepared by the same process as described in Reference Example 1 was poured at a speed of 600 g per minute into a centrifugal filter rotating at 1700 per minute to stick crude methionine on the filter fabric. Then, the number of rotation was set to 3800 per minute and water was shaken off for 2 minutes. Then, the number of rotation was set to 280 per minute and washing was performed by spraying 74 g of the reused wash water (the formulation is shown in Table 1) prepared in Reference Example 2 from a nozzle. After having disappeared the wash water discharged from the bottom of the centrifugal filter, the number of rotation was set to 3800 per minute and water was shaken off for 2 minutes. Then, the number of rotation was set to 280 per minute again and washing was performed by spraying 43 g of the additional wash water (the formulation is shown in Table 2) from a nozzle. After having disappeared the wash water discharged from the bottom of the centrifugal filter, the number of rotation was set to 3800 per minute and water was shaken off for 2 minutes. The cake remained on the filter fabric was refined methionine (46.2 g) and the content of pure methionine was 41.9 g (conversion based on HPLC measurement; yield from Reference Example 1 was 85.5%). The components comprised in refined methionine is shown in Table 3 (determined by HPLC measurement).

### Comparative Example

Crude methionine was stuck in the same process as described in Example. Then, the number of rotation was set to 3800 per minute and water was shaken off for 2 minutes. The number of rotation was set to 280 per minute and washing was performed by spraying 117 g of pure water from a nozzle. After having disappeared the wash water discharged from the bottom of the centrifugal filter, the number of rotation was set to 3800 per minute and water was shaken off for 2 minutes. The cake remained on the filter fabric was refined methionine (48.9 g) and the content of pure methionine was 41.0 g (conversion based on HPLC measurement; yield from Reference Example 1 was 83.7%). The components contained in refined methionine is shown in Table 3 (determined by HPLC measurement).

**Table 1**

| Formulation of reused wash water | |
|---|---|
| Components | % by weight |
| methionine | 3.95 |
| methionine dimer | 0.24 |
| glycine | 0.02 |
| alanine | 0.04 |
| potassium | 1.64 |

**Table 2**

| Formulation of additional wash water | |
|---|---|
| Components | % by weight |
| methionine | 3.00 |
| water | 97.00 |

**Table 3**

| Concentration of components contained in crude/refined methionine | | | | |
|---|---|---|---|---|
| | Components | Concentration of components % | | |
| | | crude | refined | after dried |
| Compara tive Ex ample | methionine | 82.63 | 83.84 | 98.76 |
| | methionine dimer | 0.39 | 0.21 | 0.25 |
| | glycine | 0.02 | - | |
| | alanine | 0.06 | 0.03 | 0.04 |
| | potassium | 1.37 | 0.08 | 0.09 |
| Example | methionine | 82.63 | 90.69 | 99.64 |
| | methionine dimer | 0.39 | 0.18 | 0.20 |
| | glycine | 0.02 | - | |
| | alanine | 0.06 | 0.03 | 0.03 |
| | potassium | 1.37 | 0.06 | 0.07 |

### Industrial Applicability

According to the present invention, it is possible to reduce the loss of methionine due to washing since washing of crude methionine is performed with the use of a methionine-containing wash water, and thus, it is possible to increase the yield of refined methionine.

In addition, the methionine-containing wash water after being used for washing crude methionine is recovered and reused, and thus, methionine contained in the wash water is efficiently utilized as methionine for a wash water. There is no need for wastewater treatment. Furthermore, the amount of a methionine-containing wash water which is newly prepared is reduced. These lead to cost saving.

Furthermore, the process can provide refined methionine in high quality since the quality of refined methionine, which is obtained by washing crude methionine with the reuse of the recovered and methionine-containing wash water, is equal to that of refined methionine obtained by washing with the use of pure water.

## Claims

1. A process for producing refined methionine from crude methionine, comprising a step of washing crude methionine with the use of a wash water containing methionine.

2. The process according to claim 1, wherein the concentration of methionine in the wash water is 1.0% by weight or higher.

3. The process according to claim 1 or 2, wherein the wash water after being used for washing crude methionine is recovered and reused.

4. The process according to claim 3, wherein washing of crude methionine is performed with the use of an additional wash water together with the reuse of a part of the wash water after being used for washing crude methionine.

5. The process according to claim 4, wherein washing of crude methionine is performed by washing with the reused wash water, followed by the washing with the additional wash water.

6. The process according to claim 4 or 5, wherein the additional wash water contains methionine.

7. The process according to claim 6, wherein the concentration of methionine in the additional wash water is 2.0% by weight or higher.

8. The process according to claim 6, wherein the additional wash water is substantially free from alkali.

9. The process according to any one of claims 6 to 8, further comprising a step of drying refined methionine, wherein the additional wash water is prepared by dissolving dried and refined methionine obtained in the step in pure water.
